# EUROPEAN PATENT SPECIFICATION

(11) **EP 1 635 123 B1**
(45) Date of publication and mention of the grant of the patent: **10.10.2007**
(21) Application number: 05255597.6
(22) Date of filing: 13.09.2005
(51) Int. Cl.: F24F 3/16, A61L 9/22, B03C 3/017

(54) **Surface discharge type air cleaning device**
Luftreinigungsvorrichtung vom Oberflächenentladungstyp
Dispositif de purification d'air du type à décharge de surface

(30) Priority: 14.09.2004 KR 2004073484
(43) Date of publication of application: 15.03.2006
(73) Proprietor: LG Electronics, Inc., Seoul 150-010 (KR)
(72) Inventor: Kim, Ho Jung, Inchun-si 403-020 (KR); Choi, In Ho, Kyungki-do 435-040 (KR); Yum, Kwan Ho, Seoul 152-050 (KR); Choi, Ho Seon, Seoul 156-090 (KR)
(74) Representative: Camp, Ronald

(56) References cited:
- WO-A-03/013620
- WO-A-20/04023615
- US-A1- 2004 145 853
- PATENT ABSTRACTS OF JAPAN vol. 2002, no. 07, 3 July 2002 (2002-07-03) & JP 2002 065838 A (SHARP CORP), 5 March 2002 (2002-03-05)
- PATENT ABSTRACTS OF JAPAN vol. 2003, no. 12, 5 December 2003 (2003-12-05) & JP 2004 103257 A (SHARP CORP), 2 April 2004 (2004-04-02)
- PATENT ABSTRACTS OF JAPAN vol. 1996, no. 12, 26 December 1996 (1996-12-26) & JP 08 217412 A (TOTO LTD), 27 August 1996 (1996-08-27)

## Description

The present invention relates to a surface discharge type air cleaning device mounted in an air conditioner for cleaning air. It more particularly relates to a surface discharge type air cleaning device wherein a discharge electrode and a ground electrode are disposed on one surface of a dielectric body such that the discharge electrode and the ground electrode are opposite to each other, whereby the service life of the surface discharge type air cleaning device is increased, and air cleaning efficiency is improved.

A prior art surface discharge type air cleaning device (see for example patent document US-A-2004/0145853) adopts a surface discharge plasma chemical processing method. Specifically, the surface discharge type air cleaning device of the prior art is a ceramic-based high frequency discharge type air cleaning device that is capable of generating a large number of hydroxyl radicals and a large amount of ozone through the formation of a strong plasma area on the surface of an element and processing noxious gases through the use of the generated hydroxyl radicals and ozone.

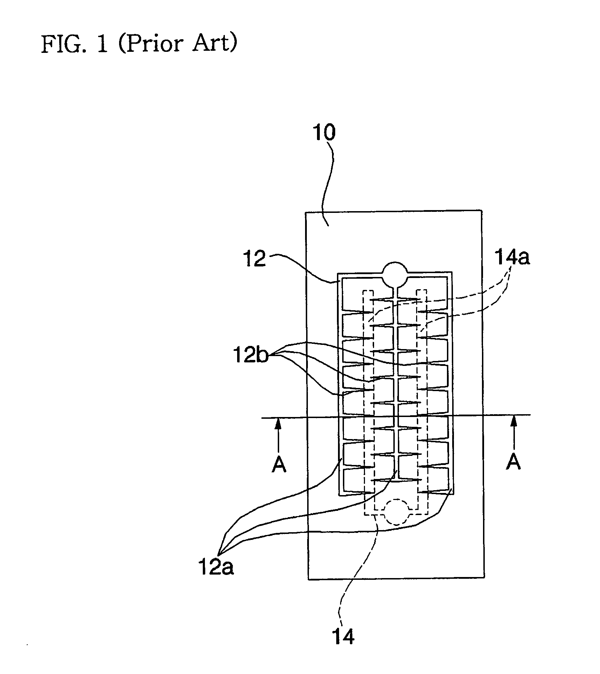

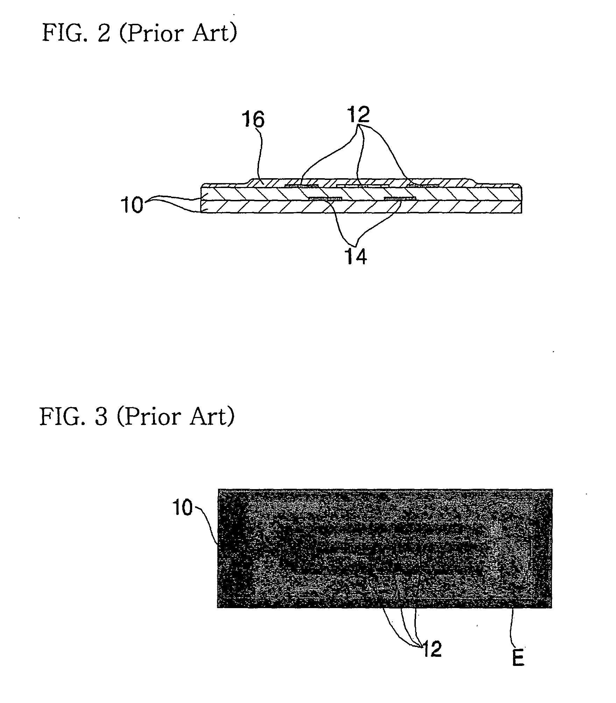

FIG. 1 is a plan view showing a conventional surface discharge type air cleaning device, and FIG. 2 is a cross-sectional view of the conventional surface discharge type air cleaning device seen from line A-A of FIG. 1.

As shown in FIGS. 1 and 2, the conventional surface discharge type air cleaning device comprises: an insulating dielectric body 10, which is composed of two rectangular sheets attached to each other while being disposed in surface contact with each other; a discharge electrode 12 disposed on the upper surface of the insulating dielectric body 10; and a ground electrode 14 disposed between the two rectangular sheets of the insulating dielectric body 10. On the upper surface of the insulating dielectric body 10 is applied a coating layer 16 for covering the discharge electrode 12 such that the discharge electrode 12 is not directly exposed to the atmosphere.

The insulating dielectric body 10 is made of a ceramic material. The discharge electrode 12 is connected to one terminal of a power source supply unit, and the ground electrode 14 is connected to the other terminal of the power source supply unit, such that the power source is supplied to not only the discharge electrode 12 but also the ground electrode 14. An alternating current power source is used as the power source.

The discharge electrode 12 comprises: three main electrodes 12a, which are arranged in parallel with one another; and subsidiary electrodes 12b protruding from the main electrodes 12a, each of the subsidiary electrodes 12b having a pointed end. The ground electrode 14 comprises: two branched ground electrodes 14a, which are arranged in parallel with each other and disposed opposite to the subsidiary electrodes 12b.

When a power source having a voltage higher than onset voltage is applied to the discharge electrode 12 and the ground electrode 14 of the conventional surface discharge type air cleaning device with the above-stated construction, a dielectric breakdown phenomenon occurs between the discharge electrode 12 and the ground electrode 14. As a result, a discharge phenomenon occurs on the surface of the insulating dielectric body 10, as shown in FIG. 3, and therefore, a strong plasma area is formed on the surface of the insulating dielectric body 10.

When the plasma is discharged as described above, a conductive path, which is called a streamer, is formed on the surface of the insulating dielectric body 10, and a large number of high-energy electrons are generated through the streamer. The high-energy electrons react with gases surrounding the high-energy electrons due to electron collision. As a result, a large amount of ozone and a large number of hydroxyl radicals and negative ions are generated.

The generated ozone, hydroxyl radicals, and negative ions oxidize and decompose pollutants, such as noxious gases contained in air, to clean the air.

As described above, the conventional surface discharge type air cleaning device performs discharge through the entire surface of the insulating dielectric body 10, and therefore, the onset voltage of the conventional surface discharge type air cleaning device is lower than that of a corona discharge type air cleaning device. Consequently, power consumption is low, and noise generated from the conventional surface discharge type air cleaning device is small, and therefore, air is efficiently cleaned by the conventional surface discharge type air cleaning device even when the conventional surface discharge type air cleaning device is used in a small space.

In the conventional surface discharge type air cleaning device, however, electric charge is easily concentrated at the subsidiary electrodes 12b, as shown in FIG. 3. Moreover, the electrical charge concentration is increased at the end part E of the discharge electrode 12. Consequently, it is required that the onset voltage and the input energy be raised in order to accomplish uniform generation distribution of streamer throughout the entire region of the dielectric body. Thermal stress is partially increased at the end part E of the discharge electrode 12, and therefore, gases surrounding the discharge electrode 12 are heated. As a result, the amount of ozone generated is increased. On the other hand, the number of hydroxyl radicals and negative ions is decreased. Also, partial deterioration of the electrode occurs rapidly due to partial increase of thermal stress, and therefore, the service life of the surface discharge type air cleaning device is shortened, and discharge safety is also lowered. Consequently, air cleaning efficiency is decreased.

Furthermore, the insulating dielectric body 10 of the conventional surface discharge type air cleaning device is composed of two sheets, between which the ground electrode 14 is disposed, and the discharge electrode is disposed on the upper surface of the insulating dielectric body 10. Consequently, the structure of the conventional surface discharge type air cleaning device is complicated, and therefore, manufacturing costs of the conventional surface discharge type air cleaning device are increased.

The present invention seeks to provide an improved surface discharge type air cleaning device.

One aspect of the present invention provides a surface discharge type air cleaning device comprising: an insulating dielectric body formed in the shape of a sheet; and a discharge electrode and a ground electrode formed at one surface of the insulating dielectric body, the discharge electrode and the ground electrode being disposed opposite to each other while the discharge electrode and the ground electrode are spaced a predetermined distance from each other.

The discharge electrode and the ground electrode may be arranged in pairs while being disposed in parallel with each other.

The air cleaning device may further comprise: a protective film applied to the surface of the insulating dielectric body for protecting the discharge electrode and the ground electrode.

The discharge electrode and the ground electrode may be provided with terminal parts, which are exposed outside the protective film and connected to an external circuit, respectively.

Another aspect of the present invention, provides a surface discharge type air cleaning device comprising: an insulating dielectric body formed in the shape of a sheet; and a discharge electrode and a ground electrode formed at one surface of the insulating dielectric body, the discharge electrode and the ground electrode being disposed in parallel with each other while the discharge electrode and the ground electrode are spaced a predetermined distance from each other, wherein the discharge electrode has a plurality of pointed ends protruding toward the ground electrode.

The pointed ends may be formed in the shape of a triangle.

A DC power source may be applied to the discharge electrode and the ground electrode.

The discharge electrode and the ground electrode may be arranged in pairs while being disposed in parallel with each other.

The air cleaning device may further comprise: a protective film applied to the surface of the insulating dielectric body for protecting the discharge electrode and the ground electrode.

Yet another aspect of the present invention, provides a surface discharge type air cleaning device comprising: an insulating dielectric body formed in the shape of a sheet; a discharge electrode and a ground electrode formed at one surface of the insulating dielectric body, the discharge electrode and the ground electrode being disposed in parallel with each other while the discharge electrode and the ground electrode are spaced a predetermined distance from each other; and a DC voltage applying circuit for applying DC voltage to the discharge electrode and the ground electrode.

Electrical charge accumulation can be prevented and continuous discharge may be possible, even when a DC power source is used. In addition, entirely uniform and stable discharge at the surface of the dielectric body may be accomplished, and therefore, the generated number of hydroxyl radicals and negative ions can be increased while the generated amount of ozone is decreased. Also, discharge safety can be increased, and therefore, air cleaning efficiency can be improved.

Embodiments of the invention will now be described, by way of non-limiting example only, with reference to the drawings, in which:
FIG. 1 is a plan view showing a conventional surface discharge type air cleaning device;
FIG. 2 is a cross-sectional view of the conventional surface discharge type air cleaning device seen from line A-A of FIG. 1;
FIG. 3 is a reference view illustrating plasma discharge of the conventional surface discharge type air cleaning device;
FIG. 4 is a perspective view of a surface discharge type air cleaning device according to a first embodiment of the present invention showing the upper surface of the surface discharge type air cleaning device;
FIG. 5 is a cross-sectional view of the surface discharge type air cleaning device according to the first embodiment of the present invention seen from line B-B of FIG. 4;
FIG. 6 is a perspective view of a surface discharge type air cleaning device according to a second embodiment of the present invention showing the upper surface of the surface discharge type air cleaning device; and
FIG. 7 is a longitudinal sectional view showing an indoor unit of an air conditioner, to which the surface discharge type air cleaning device according to the present invention is applied.

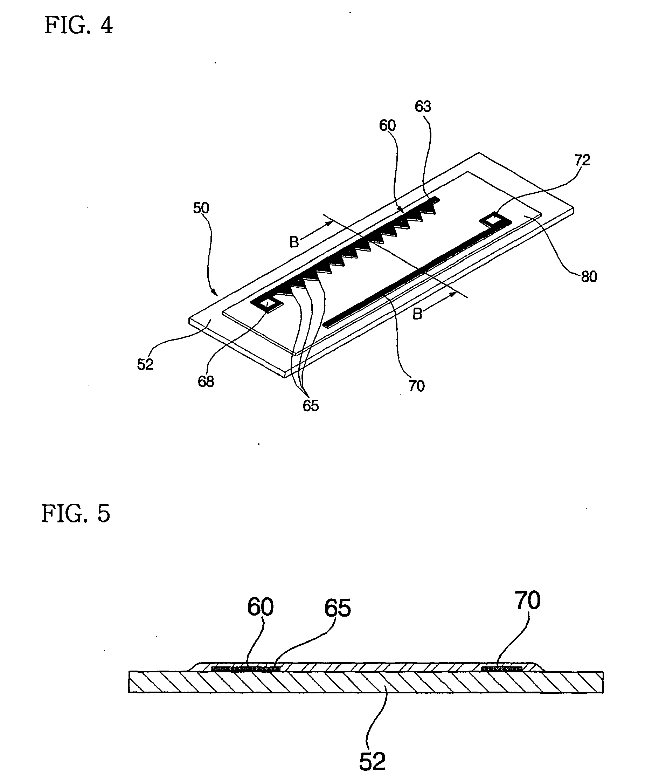

As shown in FIGS. 4 and 5, a surface discharge type air cleaning device 50 comprises: an insulating dielectric body 52; a discharge electrode 60 formed at the upper surface of the insulating dielectric body 52; and a ground electrode 70 also formed at the upper surface of the insulating dielectric body 52, the discharge electrode 60 and the ground electrode 70 being disposed in parallel with each other while being opposite to each other. The discharge electrode 60 and the ground electrode 70 are protected by a protective film 80 coated on the upper surface of the insulating dielectric body 52.

The insulating dielectric body 52 is composed of a single rectangular sheet having a predetermined thickness, which is distinguished from the insulating dielectric body of the conventional surface discharge type air cleaning device as described above. In this embodiment, the insulating dielectric body 52 is made of a resin material having high oxidization resistance for organic matter or a ceramic material for inorganic matter. However, the material of the insulating dielectric body 52 is not limited to the resin material or the ceramic material, and the shape of the insulating dielectric body 52 is not limited to the rectangular shape. The insulating dielectric body 52 may be formed of various materials and shapes according to the design conditions of the insulating dielectric body 52.

The discharge electrode 60 is formed of a pattern of a conductive metallic material printed on one side part of the upper surface of the insulating dielectric body 52.

The discharge electrode 60 comprises: a main electrode 63 formed in a linear structure, the main electrode 63 being disposed in parallel with the ground electrode 70; and a plurality of pointed ends 65 protruding from the main electrode 63 toward the ground electrode 70.

The pointed ends 64 are formed in the shape of a triangle, and the triangular pointed ends 64 are continuously connected to one another.

The ground electrode 70 is formed of a pattern of a conductive metallic material printed on the other side part of the upper surface of the insulating dielectric body 52 in the same fashion as the discharge electrode 60. The ground electrode 70 is formed in the same linear structure as the main electrode 63 of the discharge electrode 60.

The protective film 80 is made of a non-conductive material. In this embodiment, the protective film 80 is made of a material that is not easily deteriorated, and thus, not damaged when plasma is discharged through the entire surface of the insulating dielectric body 52. The protective film 80 is formed in the shape of a rectangle having a size sufficient to cover the discharge electrode 60 and the ground electrode 70. The protective film 80 is applied to the upper surface of the insulating dielectric body 52.

The protective film 80 has a partially-opened structure such that the discharge electrode 60 and the ground electrode 70 are provided with terminal parts 68 and 72, which are connected to an external circuit, respectively.

The power source applied through the terminal part 68 of the discharge electrode 60 and the terminal part 72 of the ground electrode 70 may be an alternating current (AC) power source or a direct current (DC) power source. When a DC power source is applied to the surface discharge type air cleansing device 50, a DC voltage applying circuit is connected to the surface discharge type air cleansing device 50.

DC voltage applying circuits, for applying a DC power source to an air cleaning device, are well-known in the art and therefore a detailed description thereof will not be given.

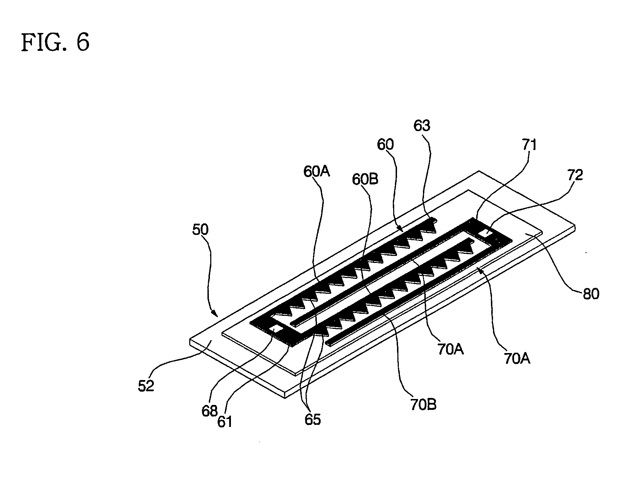

FIG. 6 is a perspective view of a surface discharge type air cleaning device 50. Components of the surface discharge type air cleaning device according to the second embodiment which are identical or similar in construction to those of the surface discharge type air cleaning device according to the first embodiment are indicated by the same reference numerals as those of the surface discharge type air cleaning device according to the first embodiment and a detailed description thereof will not be given.

Referring to FIG. 6, the surface discharge type air cleaning device 50 comprises: an insulating dielectric body 52; a pair of discharge electrodes 60A and 60B formed at the upper surface of the insulating dielectric body 52; and a pair of ground electrodes 70A and 70B also formed at the upper surface of the insulating dielectric body 52.

As shown in FIG. 6, the discharge electrodes 60A and 60B and the ground electrodes 70A and 70B are arranged in parallel with one another in the order of the discharge electrode 60A, the ground electrode 70A, the discharge electrode 60B, and the ground electrode 70B. At this time, a plurality of pointed ends 65 formed at the discharge electrodes 60A and 60B are protruded toward the corresponding ground electrodes 70A and 70B.

The discharge electrodes 60A and 60B are electrically connected to each other via a connection electrode 61. Similarly, the ground electrodes 70A and 70B are electrically connected to each other via a connection electrode 71. The connection electrodes 61 and 71 are provided with terminal parts (68, 72), which are connected to an external circuit, respectively.

In this embodiment, the discharge electrodes 60A and 60B and the ground electrodes 70A and 70B are provided in pairs. The number of the discharge electrodes 60A and 60B and the ground electrodes 70A and 70B in pairs may be changed without limits based on the size and other design conditions of the insulating dielectric body 52.

Now, the operation of the surface discharge type air cleansing device 50 with the above-stated construction will be described in detail.

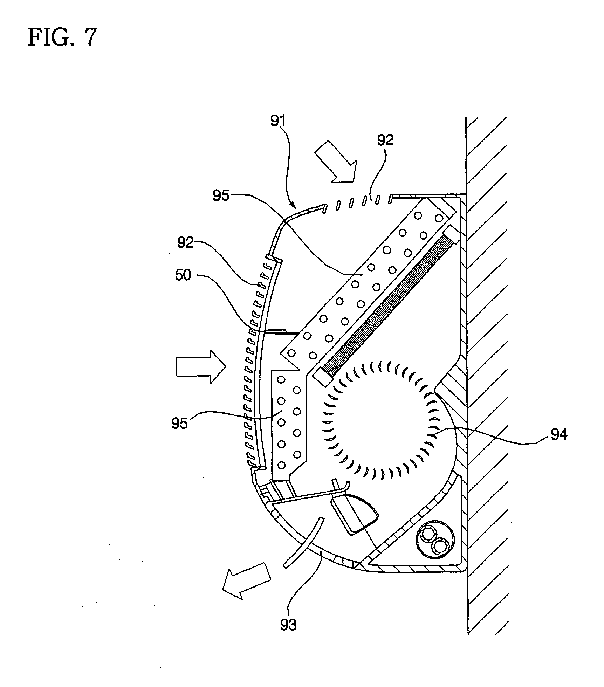

FIG. 7 is a longitudinal sectional view showing an indoor unit 91 of an air conditioner, to which the surface discharge type air cleaning device 50.

Generally, the indoor unit 91 of the air conditioner is provided with an inlet port 92 and an outlet port 93, through which indoor air is circulated. In the indoor unit 91 are mounted a blower 94 for forcibly circulating air and a heat exchanger 95 for performing heat exchange with air passing through the heat exchanger 95.

The surface discharge type air cleaning device 50 may be disposed at any position on an air channel in the indoor unit. In this embodiment, the surface discharge type air cleaning device 50 is disposed inside the inlet port 92. The surface discharge type air cleaning device 50 is formed in the shape of a sheet, and therefore, the surface discharge type air cleaning device 50 is preferably disposed in parallel with the air flow direction such that flow resistance is minimized.

In the illustrated embodiment, only one surface discharge type air cleaning device 50 is mounted in the indoor unit 91. In a modification not shown, several surface discharge type air cleaning devices may be mounted in the indoor unit 91 if necessary.

The operation of the surface discharge type air cleaning device 50 according to the present invention will be described hereinafter in detail under the condition that the surface discharge type air cleaning device 50 is mounted in the indoor unit 91 as described above.

When the air conditioner is turned on to operate the blower 94, indoor air is introduced into the indoor unit 91 through the inlet port 92 and passes through the heat exchanger. As a result, the air is cooled, and is then discharged into the interior of a room where the indoor unit 91 is installed. When a power source is applied to the surface discharge type air cleaning device 50 to clean the indoor air, some of the air introduced into the indoor unit 91 through the inlet port 92 passes by the surface discharge type air cleaning device 50. As a result, pollutants are sterilized or decomposed, and therefore, the air is cleaned.

Specifically, when the air conditioner is operated, and the power source having voltage greater than onset voltage is applied to the discharge electrode 60 and the ground electrode 70, a dielectric breakdown phenomenon occurs at the surface of the insulating dielectric body 52 between the discharge electrode 60 and the ground electrode 70, and a plasma discharge area is formed on the surface of the insulating dielectric body 52. At this time, a streamer is formed on the surface of the insulating dielectric body 52. As a result, a large number of high-energy electrons are generated through the streamer, and the high-energy electrons react with gases surrounding the high-energy electrons due to electron collision. Consequently, a small amount of ozone and a large number of hydroxyl radicals and negative ions are generated.

The generated ozone, the amount of which is small, and the generated hydroxyl radicals and negative ions, the number of which is large, oxidize and decompose pollutants, such as noxious gases, contained in the indoor air, to clean the air.

The discharge electrode 60 and the ground electrode 70 are disposed on the upper surface of the insulating dielectric body 52 while being arranged in parallel with each other. Consequently, electrical charges are uniformly distributed between the discharge electrode 60 and the ground electrode 70, and therefore, stable plasma formation is possible, and generation distribution of the streamer is also uniformly accomplished.

Also, the pointed ends, which serve to generate high voltage, are protruded from the discharge electrode 60 toward the ground electrode 70. Consequently, electrical charge concentration or accumulation on the surface of the discharge electrode 60 is prevented, even though the DC power source is used as the input power source instead of the AC power source, and therefore, plasma discharge is entirely uniformly carried out in the vicinity of the pointed ends of the discharge electrode 60.

Furthermore, the surface discharge type air cleaning device 50 according to the present invention can accomplish minute discharge at lower voltage than the conventional surface discharge type air cleaning device. Consequently, a large number of hydroxyl radicals and negative ions are generated at the low voltage while the generated amount of ozone is minimized, and therefore, oxidization and decomposition of noxious gases are smoothly carried out.

In conclusion, the surface discharge type air cleaning device 50 is capable of increasing generation of hydroxyl radicals and negative ions while decreasing generation of ozone, which is toxic to humans, by lowering onset voltage and input energy. Consequently, sterilization and purification of the indoor air are carried out using the hydroxyl radicals and the negative ions. Furthermore, partial increase of thermal stress can be effectively prevented, and therefore, the service life of the surface discharge type air cleaning device can be increased, and discharge safety can be improved.

In the above description, the surface discharge type air cleaning device 50 is applied to the indoor unit of the air conditioner. However, the surface discharge type air cleaning device may be applied to all kinds of equipment, such as various air purifiers or noxious gas purifying apparatuses.

As apparent from the above description, the surface discharge type air cleaning device has the following effects.

The discharge electrode and the ground electrode are disposed on the upper surface of the insulating dielectric body while being arranged in parallel with each other, and therefore, electrical charge is uniformly distributed between the discharge electrode and the ground electrode, and more stable plasma formation is accomplished. Consequently, the generated number of hydroxyl radicals and negative ions, which sterilize and decompose noxious gases, is increased while the generated amount of ozone, which is generated as the gases are heated, is decreased. In addition, the applied voltage is lowered, and therefore, power consumption is reduced.

Also, the pointed ends are protruded from the discharge electrode toward the ground electrode, and therefore, electrical charge accumulation is prevented and continuous discharge is possible, even when the DC power source is used. In addition, entirely uniform and stable discharge at the surface of the dielectric body is accomplished, and therefore, decrease of the service life of the surface discharge type air cleaning device due to partial deterioration of the discharge electrode is prevented, and discharge safety is increased. Consequently, air cleaning efficiency is improved.

Furthermore, the discharge electrode and the ground electrode are formed on the upper surface of the insulating dielectric body, which is composed of a single sheet. Consequently, the structure of the surface discharge type air cleaning device is simplified, and manufacturing costs of the surface discharge type air cleaning device are reduced.

Although the embodiments of the present invention have been disclosed for illustrative purposes, those skilled in the art will appreciate that various modifications, additions and substitutions are possible, without departing from the scope of the invention as defined by the appended claims.

## Claims

1. A surface discharge type air cleaning device comprising
an insulating dielectric body (52) formed in the shape of a sheet,
a discharge electrode (60) and a ground electrode (70), the air cleaning device **characterized in that** the discharge electrode (60) and the ground electrode (70) are formed at one surface of the insulating dielectric body (52), the discharge electrode (60) and the ground electrode (70) being disposed opposite to each other while the discharge electrode (60) and the ground electrode (70) are spaced a predetermined distance from each other.

2. The air cleaning device as set forth in claim 1, wherein the discharge electrode (60) and the ground electrode (70) are arranged in pairs while being disposed in parallel with each other.

3. The air cleaning device as set forth in claim 1, further comprising:
a protective film (80) applied to the surface of the insulating dielectric body (52) for protecting the discharge electrode (60) and the ground electrode (70).

4. The air cleaning device as set forth in claim 3, wherein the discharge electrode (60) and the ground electrode (70) are provided with terminal parts (68, 72), which are exposed outside the protective film (80) and connected to an external circuit, respectively.

5. The air cleaning device as set forth in claim 1, wherein the discharge electrode (60) and the ground electrode (70) being disposed in parallel with each other while the discharge electrode (60) and the ground electrode (70) are spaced a predetermined distance from each other, and wherein
the discharge electrode (60) has a plurality of pointed ends (65) protruding toward the ground electrode (70).

6. The air cleaning device as set forth in claim 5, wherein the pointed ends (64) are formed in the shape of a triangle.

7. The air cleaning device as set forth in claim 5, wherein a DC power source is applied to the discharge electrode (60) and the ground electrode (70).

8. The air cleaning device as set forth in claim 5, wherein the discharge electrode (60) and the ground electrode (70) are arranged in pairs while being disposed in parallel with each other.

9. The air cleaning device as set forth in claim 5, further comprising:
a protective film (80) applied to the surface of the insulating dielectric body (52) for protecting the discharge electrode (60) and the ground electrode (70).

10. The air cleaning device as set forth in claim 1, wherein the discharge electrode (60) and the ground electrode (70) being disposed in parallel with each other while the discharge electrode (60) and the ground electrode (70) are spaced a predetermined distance from each other, and wherein,
a DC voltage applying circuit for applying DC voltage to the discharge electrode (60) and the ground electrode (70).

## Patentansprüche

1. Luftreinigungseinrichtung des Oberflächenentladungstyps, die Folgendes aufweist:
einen blattförmig ausgebildeten isolierenden dielektrischen Körper (52);
eine Entladungselektrode (60) und eine Masseelektrode (70), die auf einer Oberfläche des isolierenden dielektrischen Körpers (52) ausgebildet sind,
wobei die Luftreinigungseinrichtung **dadurch gekennzeichnet ist, dass** die Entladungselektrode (60) und die Masseelektrode (70) einander gegenüberliegend angeordnet sind, während die Entladungselektrode (60) und die Masseelektrode (70) in einem vorbestimmten Abstand voneinander beabstandet sind.

2. Luftreinigungseinrichtung nach Anspruch 1, wobei die Entladungselektrode (60) und die Masseelektrode (70) paarweise angeordnet sind, während sie zueinander parallel ausgerichtet sind.

3. Luftreinigungseinrichtung nach Anspruch 1, die weiter Folgendes aufweist:
einen Schutzfilm (80), der auf der Oberfläche des isolierenden dielektrischen Körpers (52) aufgetragen ist, um die Entladungselektrode (60) und die Masseelektrode (70) zu schützen.

4. Luftreinigungseinrichtung nach Anspruch 3, wobei die Entladungselektrode (60) und die Masseelektrode (70) mit Endteilen (68, 72) versehen sind, die außerhalb des Schutzfilms (80) angeordnet und jeweils mit einer externen Schaltung verbunden sind.

5. Luftreinigungseinrichtung nach Anspruch 1, wobei die Entladungselektrode (60) und die Masseelektrode (70) parallel zueinander angeordnet sind, während die Entladungselektrode (60) und die Masseelektrode (70) in einem vorbestimmten Abstand voneinander beabstandet sind, und wobei die Entladungselektrode (60) eine Vielzahl zugespitzter Enden (65) aufweist, die in Richtung auf die Masseelektrode (70) verlaufen.

6. Luftreinigungseinrichtung nach Anspruch 5, wobei die zugespitzten Enden (64) in Form eines Dreiecks ausgebildet sind.

7. Luftreinigungseinrichtung nach Anspruch 5, wobei die Entladungselektrode (60) und die Masseelektrode (70) mit einer Gleichstrom-Energieversorgung beaufschlagt sind.

8. Luftreinigungseinrichtung nach Anspruch 6, wobei die Entladungselektrode (60) und die Masseelektrode (70) paarweise angeordnet sind, während sie zueinander parallel ausgerichtet sind.

9. Luftreinigungseinrichtung nach Anspruch 5, die ferner Folgendes aufweist:
einen Schutzfilm (80), der auf der Oberfläche des isolierenden dielektrischen Körpers (52) aufgetragen ist, um die Entladungselektrode (60) und die Masseelektrode (70) zu schützen.

10. Luftreinigungseinrichtung nach Anspruch 1, wobei die Entladungselektrode (60) und die Masseelektrode (70) parallel zueinander ausgerichtet sind, während die Entladungselektrode (60) und die Masseelektrode (70) in einem vorbestimmten Abstand voneinander beabstandet sind, und wobei eine Gleichspannungs-Beaufschlagungsschaltung für die Beaufschlagung der Entladungselektrode (60) und der Masseelektrode (70) mit Gleichspannung vorgesehen ist.

## Revendications

1. Dispositif d'épuration d'air de type décharge superficielle comprenant
un corps diélectrique isolant (52) formé sous la forme d'une feuille
une électrode émissive (60) et une électrode de masse (70), le dispositif d'épuration d'air étant **caractérisé en ce que** l'électrode émissive (60) et l'électrode de masse (70) sont formées sur une surface du corps diélectrique isolant (52), l'électrode émissive (60) et l'électrode de masse (70) étant disposées opposées l'une à l'autre tandis que l'électrode émissive (60) et l'électrode de masse (70) sont espacées l'une de l'autre d'une distance prédéterminée.

2. Dispositif d'épuration d'air selon la revendication 1, dans lequel l'électrode émissive (60) et l'électrode de masse (70) sont agencées en paire tout en étant disposées en parallèle l'une de l'autre.

3. Dispositif d'épuration d'air selon la revendication 1, comprenant en outre:
un film protecteur (80) appliqué à la surface du corps diélectrique isolant (52) pour protéger l'électrode émissive (60) et l'électrode de masse (70).

4. Dispositif d'épuration d'air selon la revendication 3, dans lequel l'électrode émissive (60) et l'électrode de masse (70) sont pourvues de parties terminales (68, 72), qui sont mises à nu à l'extérieur du film protecteur (80) et connectées à un circuit externe, respectivement.

5. Dispositif d'épuration d'air selon la revendication 1, dans lequel l'électrode émissive (60) et l'électrode de masse (70) sont disposées en parallèle l'une de l'autre tandis que l'électrode émissive (60) et l'électrode de masse (70) sont espacées l'une de l'autre d'une distance prédéterminée, et dans lequel
l'électrode émissive (60) comporte une pluralité d'extrémités pointues (65) faisant saillie vers l'électrode de masse (70).

6. Dispositif d'épuration d'air selon la revendication 5, dans lequel les extrémités pointues (64) sont formées sous la forme d'un triangle.

7. Dispositif d'épuration d'air selon la revendication 5, dans lequel une source de courant continu est appliquée à l'électrode émissive (60) et à l'électrode de masse (70).

8. Dispositif d'épuration d'air selon la revendication 5, dans lequel l'électrode émissive (60) et l'électrode de masse (70) sont agencées en paire tout en étant disposées en parallèle l'une de l'autre.

9. Dispositif d'épuration d'air selon la revendication 5, comprenant en outre:
un film protecteur (80) appliqué à la surface du corps diélectrique isolant (52) pour protéger l'électrode émissive (60) et l'électrode de masse (70).

10. Dispositif d'épuration d'air selon la revendication 1, dans lequel l'électrode émissive (60) et l'électrode de masse (70) sont disposées en parallèle l'une de l'autre tandis que l'électrode émissive (60) et l'électrode de masse (70) sont espacées l'une de l'autre d'une distance prédéterminée, et dans lequel un circuit d'application de tension continue est destiné à appliquer une tension continue à l'électrode émissive (60) et à l'électrode de masse (70).
